# EUROPEAN PATENT APPLICATION

(11) **EP 4 293 682 A1**
(43) Date of publication of application: **20.12.2023**
(21) Application number: 23179059.3
(22) Date of filing: 13.06.2023
(51) Int. Cl.: G16H 40/20, G16H 40/63, G06V 20/52, G16H 30/40, G06N 20/00, G06V 10/70, G06V 40/00

(54) **OPERATING ROOM MONITORING AND ALERTING SYSTEM**

(30) Priority: 14.06.2022 US 202263366396 P
(71) Applicant: Stryker Corporation, Kalamazoo, MI 49002 (US)
(72) Inventor: BHARDWAJ, Gaurav, Kalamazoo, 49002 (US); TIWARY, Vijay Kumar, Kalamazoo, 49002 (US); SHARMA, Chitrank, Kalamazoo, 49002 (US)
(74) Representative: V.O.

(57) **Abstract**

The present disclosure relates generally to improving surgery safety, and more specifically to monitoring various aspects of an operating room. An exemplary method for generating an alert to close a door of an operating room comprises: determining a status of the door of the operating room by: receiving one or more images of the door captured by one or more cameras; inputting the one or more images into a trained machine-learning model to obtain the status of the door, wherein the machine-learning model is trained using training images depicting open or closed doors; receiving one or more signals from one or more sensors in the operating room; determining, based on the one or more signals, whether an alert threshold is reached; and if the alert threshold is reached and the status of the door is open, generating the alert to close the door of the operating room.

## Description

### CROSS-REFERENCE TO RELATED APPLICATIONS

This application claims the benefit of U.S. Provisional Application No. 63/366,396, filed June 14, 2022, the entire contents of which are hereby incorporated by reference herein.

### FIELD

The present disclosure relates generally to improving surgery safety, and more specifically to techniques for monitoring various aspects of an operating room.

### BACKGROUND

Ensuring surgical safety and preventing surgical site infection ("SSI") is a major focus area for hospitals. Surgical operations are carried out in an aseptic environment such as an operating room ("OR"). However, door(s) of the operating room, if left open for a long-time during surgery, can impact the integrity of the aseptic environment. For example, the opened door may cause change in temperature, pressure, particle count, etc., which can in turn compromise the safety of the operating room and lead to SSI.

Currently, intelligent, real-time alerts are not provided to trigger appropriate actions (e.g., closing the door) to minimize risk of SSI during surgical operations. Instead, there are disparate systems working in silos and they do not send automatic real-time alerts to a user (e.g., hospital staff) to take appropriate actions such as closing the OR door. There is also limited surgery data to retroactively investigate causes for acquired SSI in a hospital.

### SUMMARY

Disclosed herein are exemplary devices, apparatuses, systems, methods, and non-transitory storage media for monitoring various aspects of the operating room and providing alerts. An exemplary system can determine a status of the door (e.g., open, closed) of the operating room by: receiving one or more images of the door captured by one or more cameras and inputting the one or more images into at least one trained machine-learning model to obtain the status of the door. The machine-learning model is trained using a plurality of training images depicting open or closed doors. The system can further receive one or more signals from one or more sensors (e.g., a temperature sensor, a humidity sensor, a pressure sensor, an air quality sensor, etc.) in the operating room and determine, based on the one or more signals, whether an alert threshold is reached. If the alert threshold is reached and the status of the door is open, the system generates the alert to close the door of the operating room. In some examples, the one or more images of the doors are provided into a plurality of trained machine-learning models to obtain the status of the door.

Optionally, the system displays the generated alert on a display in the operating room. Alternatively, or additionally, the system displays the generated alert on a display in a monitoring area (e.g., at a central control center for monitoring multiple operating rooms). The system can display the alert as a message (e.g., a text message, a notification) on a mobile device. The alert may comprise a visual component, an audio component, a haptic component, or any combination thereof. The alert can be provided based on user-configurable settings (e.g., at user-specified frequency).

Some or all of the analytics described herein can be displayed in a display inside or outside the operating room, including: the current door status, the time period during which the door remains open, the average duration that the door is open, the triggered alert thresholds (e.g., current temperature and whether it is outside a normal range, current humidity and whether it is outside a normal range, current pressure and whether it is outside a normal range, current air quality and whether it is outside a normal range), a number of times that the door is open during the surgery, an average duration the door is open, a number of times a threshold breach occurred during a surgery, etc. Air quality can, for example, be measured by measuring (e.g., using one or more sensors) the amount of one or more particular particles in the air.

The system can store some or all of the sensor readings over time, such as temperature measurements, humidity measurements, pressure measurements, and air quality measurements. The system, for example, stores some or all of the analytics described herein. The above-described information can be transmitted to electronic medical records ("EMR") systems for storage and/or further analysis. For example, the system can analyze the electronic medical record, such as the recorded sensor data, to determine a cause for a post-surgery complication (e.g., SSI). For example, the system can identify correlations between any of the data points above with the occurrence or severity of the complication. The system can be configured to analyze the electronic medical record to determine a recommended protocol change for future surgeries. For example, if the system determines a correlation between the duration of door opening and a post-surgery complication, the system may automatically formulate a new protocol requiring that the door remain closed for a certain period of time.

Accordingly, examples of the present disclosure ensure surgical safety and prevent/reduce SSI by monitoring various aspects of the operating room and providing alerts to take appropriate actions during a surgical procedure. The data gathered and analysis performed can be used to investigate causes for acquired SSI in a hospital retroactively and formulate improved protocol to ensure patient safety.

An exemplary method for generating an alert to close a door of an operating room comprises: determining a status of the door of the operating room by: receiving one or more images of the door captured by one or more cameras; inputting the one or more images into a trained machine-learning model to obtain the status of the door, wherein the machine-learning model is trained using a plurality of training images depicting open or closed doors; receiving one or more signals from one or more sensors in the operating room; determining, based on the one or more signals, whether an alert threshold is reached; and if the alert threshold is reached and the status of the door is open, generating the alert to close the door of the operating room.

According to some aspects, the method further comprises: receiving a video stream captured by the one or more cameras; and determining, based on at least a portion of the video stream (e.g., one or more image frames, a shorter video), whether a surgery is in progress; in accordance with a determination that the surgery is in progress, starting the determination of the status of the door of the operating room; in accordance with a determination that the surgery is not in progress, foregoing determining the status of the door of the operating room.

According to some aspects, determining whether the surgery is in progress comprises: detecting one or more objects in the video stream; and determining whether the surgery is in progress based on the tracked one or more objects.

According to some aspects, the one or more objects include: a stretcher, a patient, a surgical mask, an intubation mask, an anesthesia cart, a cleaning cart, an operating table, an X-Ray device, an imaging device, a surgeon, the surgeon's hand, a scalpel, an endoscope, a trocar, an oxygen mask, a light in the operating room, the door, a surgical drape, a case cart, a surgical robot, or any combination thereof.

According to some aspects, determining whether the surgery is in progress is based on: whether the stretcher with the patient is brought into the operating room, whether the surgeon is masked, whether the patient is masked, whether the patient is draped, whether the surgeon is donning a gown, whether the patient is intubated, whether the patient is on the operating table, whether an incision is made, whether the surgical light is in use, whether the X-Ray device is in use, whether the anesthesia cart is in use, whether the imaging device is in use or within a predefined proximity to the patient, whether the cleaning cart is in use, whether the case cart has been brought into the operating room, whether one or more instruments from the case cart are unwrapped, or any combination thereof.

According to some aspects, the trained machine-learning model is an object detection algorithm.

According to some aspects, the trained machine-learning model is a neural network model.

According to some aspects, the machine-learning model is trained using a plurality of annotated images.

According to some aspects, the one or more sensors include: a temperature sensor, a humidity sensor, a pressure sensor, an air quality sensor, a gas sensor, or any combination there.

According to some aspects, the one or more sensors are placed within a predefined distance from a surgery table.

According to some aspects, the alert threshold includes a temperature threshold, a humidity threshold, a pressure threshold, an air quality threshold, or any combination thereof.

According to some aspects, the one or more cameras include a camera integrated into a surgical light.

According to some aspects, the method further comprises: displaying the generated alert on a display in the operating room.

According to some aspects, the method further comprises: displaying the generated alert on a display in a monitoring area.

According to some aspects, the method further comprises: displaying the alert as a message on a mobile device. According to some aspects, the system may send an electronic notification (e.g., an email, an audio message).

According to some aspects, the method further comprises: storing the determined door status, an amount of time that the door is open during a surgery, a number of times that the door is open during the surgery, an average duration the door is open, a number of times a threshold breach occurred during a surgery, and/or the one or more signals as part of an electronic medical record.

According to some aspects, the method further comprises: analyzing the electronic medical record to determine a cause for a post-surgery complication.

According to some aspects, the method further comprises: analyzing the electronic medical record to determine a recommended protocol change for future surgeries.

According to some aspects, the door of the operating room is to a non-sterile corridor where patient enters/exits through.

According to some aspects, the door of the operating room is to a sterile room where sterile equipment and staff enter/exit through.

According to some aspects, details regarding the type of door (e.g., door to non-sterile corridor, door to a sterile room) are recorded in the EMR. According to some aspects, the system determines whether to issue an alert and/or what the alert should be based on the door type. For instance, it may be common for the door to the sterile room to open a few times during the surgery. But once a surgical milestone is reached (e.g., surgery has started), opening of the door to the non-sterile corridor may be considered a "never" event. Thus, the threshold for issuing an alert to close the door to the non-sterile corridor may be lower than the threshold for issuing an alert to close the door to the sterile room. For example, the system may issue an alert to close the door to the non-sterile corridor whenever it is open during the surgery, but only issue an alert to close the door to the sterile room when the door has been opened for more than a time period or a number of times and/or when the sensor readings in the operating room exceed predefined thresholds.

An exemplary system for generating an alert to close a door of an operating room comprises: one or more processors; a memory; and one or more programs, wherein the one or more programs are stored in the memory and configured to be executed by the one or more processors, the one or more programs including instructions for: determining a status of the door of the operating room by: receiving one or more images of the door captured by one or more cameras; inputting the one or more images into a trained machine-learning model to obtain the status of the door, wherein the machine-learning model is trained using a plurality of training images depicting open or closed doors; receiving one or more signals from one or more sensors in the operating room; determining, based on the one or more signals, whether an alert threshold is reached; and if the alert threshold is reached and the status of the door is open, generating the alert to close the door of the operating room.

According to some aspects, the one or more programs further comprise instructions for: receiving a video stream captured by the one or more cameras; and determining, based on at least a portion of the video stream, whether a surgery is in progress; in accordance with a determination that the surgery is in progress, starting the determination of the status of the door of the operating room; in accordance with a determination that the surgery is not in progress, foregoing determining the status of the door of the operating room.

According to some aspects, determining whether the surgery is in progress comprises: detecting one or more objects in the video stream; and determining whether the surgery is in progress based on the tracked one or more objects.

According to some aspects, the one or more objects include: a stretcher, a patient, a surgical mask, an intubation mask, an anesthesia cart, a cleaning cart, an operating table, an X-Ray device, an imaging device, a surgeon, the surgeon's hand, a scalpel, an endoscope, a trocar, an oxygen mask, a light in the operating room, the door, a surgical drape, a case cart, a surgical robot, or any combination thereof.

According to some aspects, determining whether the surgery is in progress is based on: whether the stretcher is brought into the operating room, whether the surgeon is masked, whether the patient is masked, whether the patient is draped, whether the surgeon is donning a gown, whether the patient is intubated, whether the patient is on the operating table, whether an incision is made, whether the surgical light is in use, whether the X-Ray device is in use, whether the anesthesia cart is in use, whether the imaging device is in use or within a predefined proximity to the patient, whether the cleaning cart is in use, whether the case cart has been brought into the operating room, whether one or more instruments from the case cart are unwrapped, or any combination thereof.

According to some aspects, the trained machine-learning model is an object detection algorithm.

According to some aspects, the trained machine-learning model is a neural network model.

According to some aspects, the machine-learning model is trained using a plurality of annotated images.

According to some aspects, the one or more sensors include: a temperature sensor, a humidity sensor, a pressure sensor, an air quality sensor, a gas sensor, or any combination there.

According to some aspects, the one or more sensors are placed within a predefined distance from a surgery table.

According to some aspects, the alert threshold includes a temperature threshold, a humidity threshold, a pressure threshold, an air quality threshold, or any combination thereof.

According to some aspects, the one or more cameras include a camera integrated into a surgical light.

According to some aspects, the one or more programs further comprise instructions for: displaying the generated alert on a display in the operating room.

According to some aspects, the one or more programs further comprise instructions for: displaying the generated alert on a display in a monitoring area (e.g., a central monitoring room, a nurse station in the corridor)

According to some aspects, the one or more programs further comprise instructions for: displaying the alert as a message on a mobile device.

According to some aspects, the one or more programs further comprise instructions for: storing the determined door status, an amount of time that the door is open during a surgery, a number of times that the door is open during the surgery, an average duration the door is open, a number of times a threshold breach occurred during a surgery, and/or the one or more signals as part of an electronic medical record.

According to some aspects, the one or more programs further comprise instructions for: analyzing the electronic medical record to determine a cause for a post-surgery complication.

According to some aspects, the one or more programs further comprise instructions for: analyzing the electronic medical record to determine a recommended protocol change for future surgeries.

According to some aspects, the door of the operating room is to a non-sterile corridor where patient enters/exits through.

According to some aspects, the door of the operating room is to a sterile room where sterile equipment and staff enter/exit through.

According to some aspects, the method further comprises: if the alert threshold is reached and the status of the door is closed: foregoing generating the alert to close the door of the operating room; and generating an environmental alert.

According to some aspects, the one or more programs further comprise instructions for: if the alert threshold is reached and the status of the door is closed: foregoing generating the alert to close the door of the operating room; and generating an environmental alert.

An exemplary non-transitory computer-readable storage medium stores one or more programs, the one or more programs comprising instructions, which when executed by one or more processors of an electronic device, cause the electronic device to perform any of the methods described herein.

### BRIEF DESCRIPTION OF THE FIGURES

FIG. 1 illustrates an exemplary view of a medical care area.
FIG. 2 illustrates an exemplary process for generating an alert to close a door of an operating room.
FIG. 3A illustrates an exemplary machine-learning model used to detect surgical milestones.
FIG. 3B illustrates an exemplary machine-learning model used to detect objects and/or events, which are in turn used to detect surgical milestones.
FIG. 4A illustrates an exemplary machine-learning model used to determine the status of the door.
FIG. 4B illustrates an exemplary training process.
FIG. 5 depicts an exemplary electronic device.

### DETAILED DESCRIPTION

Disclosed herein are exemplary devices, apparatuses, systems, methods, and non-transitory storage media for monitoring various aspects of the operating room and providing alerts. An exemplary system can determine a status of the door (e.g., open, closed) of the operating room by: receiving one or more images of the door captured by one or more cameras; and inputting the one or more images into a trained machine-learning model to obtain the status of the door. The machine-learning model is trained using a plurality of training images depicting open or closed doors. The system can further receive one or more signals from one or more sensors (e.g., a temperature sensor, a humidity sensor, a pressure sensor, an air quality sensor, etc.) in the operating room and determine, based on the one or more signals, whether an alert threshold is reached. If the alert threshold is reached and the status of the door is open, the system generates the alert to close the door of the operating room.

The system can display the generated alert on a display in the operating room. Alternatively, or additionally, the system can display the generated alert on a display in a monitoring area (e.g., at a central control center for monitoring multiple operating rooms). Alternatively, or additionally, the system can display the alert as a message (e.g., a text message, a notification) on a mobile device. The alert may comprise a visual component, an audio component, a haptic component, or any combination thereof. The alert can be provided based on user-configurable settings (e.g., at user-specified frequency).

Some or all of the analytics described herein can be displayed in a display inside or outside the operating room, including: the current door status, the time period during which the door remains open, the average duration the door is open, the triggered alert thresholds (e.g., current temperature and whether it is outside a normal range, current humidity and whether it is outside a normal range, current pressure and whether it is outside a normal range, current air quality and whether it is outside a normal range), a number of times that the door is open during the surgery, an average duration the door is open, a number of times a threshold breach occurred during a surgery, the number of times the door to the sterile room has been opened during the surgery, the number of times the door to the non-sterile corridor has been opened during the surgery, the average time the door to the sterile room remains open when it is opened, the average time the door to the non-sterile corridor remains open when it is opened, etc. If the door open counts exceed one or more configurable thresholds (which may be case duration specific or surgery type specific), the system can, for example, issue an alert to recommend some additional infection prevention protocol (e.g., increased antibiotic dose, or longer course of antibiotic treatment, or use of a different antibiotic, or use of a supplemental antibiotic.

The system can store some or all of the sensor readings over time, such as temperature measurements, humidity measurements, pressure measurements, and air quality measurements. In some examples, the system stores some or all of the analytics described herein. The above-described information can be transmitted to electronic medical records ("EMR") systems for storage and/or further analysis. For example, the system can analyze the electronic medical record to determine a cause for a post-surgery complication (e.g., SSI). For example, the system can identify correlations between any of the data points above with the occurrence or severity of the complication. The system can analyze the electronic medical record to determine a recommended protocol change for future surgeries. For example, if the system determines a correlation between the duration of door opening and a post-surgery complication, the system may automatically formulate a new protocol requiring that the door remain closed for a certain period of time.

Accordingly, examples of the present disclosure ensures surgical safety and prevents/reduces SSI by monitoring various aspects of the operating room and providing alerts to take appropriate actions during a surgical procedure. The data gathered and analysis performed can be used to investigate causes for acquired SSI in a hospital retroactively and formulate improved protocols to ensure patient safety.

The following description sets forth exemplary methods, parameters, and the like. It should be recognized, however, that such description is not intended as a limitation on the scope of the present disclosure but is instead provided as a description of exemplary examples.

Although the following description uses terms "first," "second," etc., to describe various elements, these elements should not be limited by the terms. These terms are only used to distinguish one element from another. For example, a first graphical representation could be termed a second graphical representation, and, similarly, a second graphical representation could be termed a first graphical representation, without departing from the scope of the various described examples. The first graphical representation and the second graphical representation are both graphical representations, but they are not the same graphical representation.

The terminology used in the description of the various described examples herein is for the purpose of describing particular examples only and is not intended to be limiting. As used in the description of the various described examples and the appended claims, the singular forms "a," "an," and "the" are intended to include the plural forms as well, unless the context clearly indicates otherwise. It will also be understood that the term "and/or" as used herein refers to and encompasses any and all possible combinations of one or more of the associated listed items. It will be further understood that the terms "includes," "including," "comprises," and/or "comprising," when used in this specification, specify the presence of stated features, integers, steps, operations, elements, and/or components, but do not preclude the presence or addition of one or more other features, integers, steps, operations, elements, components, and/or groups thereof.

The term "if' is, optionally, construed to mean "when," "upon," "in response to determining," or "in response to detecting," depending on the context. Similarly, the phrase "if it is determined" or "if [a stated condition or event] is detected" is, optionally, construed to mean "upon determining," "in response to determining," "upon detecting [the stated condition or event]," or "in response to detecting [the stated condition or event]," depending on the context.

Certain aspects of the present disclosure include process steps and instructions described herein in the form of an algorithm. It should be noted that the process steps and instructions of the present disclosure could be embodied in software, firmware, or hardware and, when embodied in software, could be downloaded to reside on and be operated from different platforms used by a variety of operating systems. Unless specifically stated otherwise as apparent from the following discussion, it is appreciated that, throughout the description, discussions utilizing terms such as "processing," "computing," "calculating," "determining," "displaying," "generating," or the like, refer to the action and processes of a computer system, or similar electronic computing device, that manipulates and transforms data represented as physical (electronic) quantities within the computer system memories or registers or other such information storage, transmission, or display devices.

The present disclosure, in some examples, also relates to a device for performing the operations herein. This device may be specially constructed for the required purposes, or it may comprise a general-purpose computer selectively activated or reconfigured by a computer program stored in the computer. Such a computer program may be stored in a non-transitory, computer readable storage medium, such as, but not limited to, any type of disk, including floppy disks, USB flash drives, external hard drives, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each coupled to a computer system bus. Furthermore, the computers referred to in the specification may include a single processor or may be architectures employing multiple processor designs for increased computing capability.

The methods, devices, and systems described herein are not inherently related to any particular computer or other apparatus. Various general-purpose systems may also be used with programs in accordance with the teachings herein, or it may prove convenient to construct a more specialized apparatus to perform the required method steps. The required structure for a variety of these systems will appear from the description below. In addition, the present invention is not described with reference to any particular programming language. It will be appreciated that a variety of programming languages may be used to implement the teachings of the present invention as described herein.

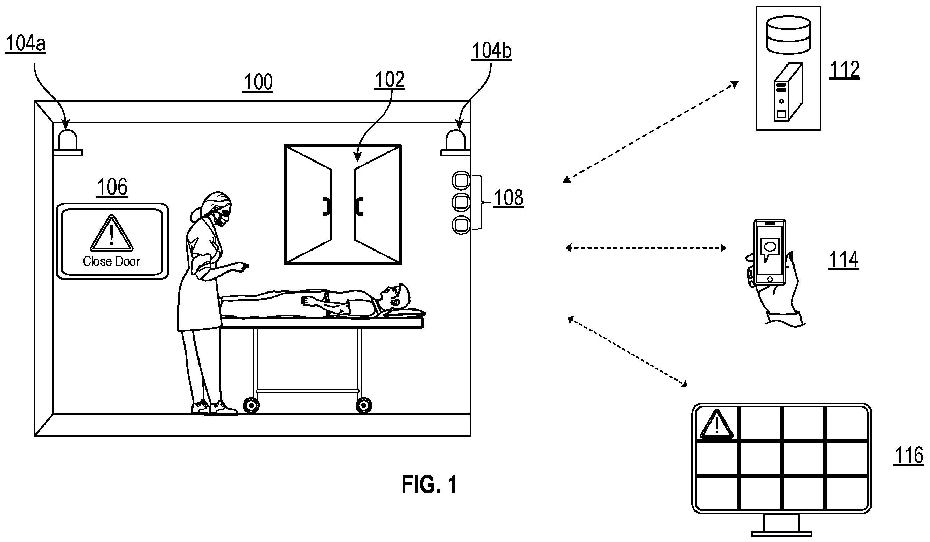

FIG. 1 illustrates an exemplary view of a medical care area 100, in accordance with some examples. In the illustrated example, the medical care area 100 is an operating room where surgical operations are carried out in an aseptic environment. The medical care area 100 includes one or more doors such as door 102. The medical care area may comprise multiple doors, for example, a door that connects to a sterile room where sterile equipment and staff enter/exit through and another door that connects to a non-sterile corridor where patient enters/exits through.

The operating room 100 in this example further comprises a plurality of sensors 108 for monitoring various aspects of the environment, such as temperature, humidity, pressure, air quality (e.g., particulate matter or particle count detected in the air), or any combination thereof. The operating room 100 further comprises a plurality of cameras such as cameras 104a and 104b. The cameras can be oriented toward one or more areas or objects of interest in the operating room. For example, one or more cameras can be oriented toward: the door such that they can capture images of the door, the operating table such that they can capture images of the operating table, a medical device (e.g., X-Ray device) such that they can capture images of the medical device, a surgical tool such that they can capture images of the surgical tool, etc. Multiple cameras can be placed in different locations in the operating room such as they can collectively capture a particular area or object of interest from different perspectives. Some cameras can track a moving object. The one or more cameras can, for example, include PTZ cameras. The cameras can include cameras that can provide a video stream over a network. The one or more cameras can, for example, include a camera integrated into a surgical light in the operating room.

Various sensors may be placed in areas of interest in the operating room to improve accuracy of the monitoring. For example, one or more sensors can be placed within a predefined distance to the operating table, the door, room air vents, etc. The total number of sensors deployed in an operating room can vary based on the size of the room. For example, if the size of the room exceeds a predefined threshold, multiple sensors of the same type can be deployed to ensure accurate monitoring of the entire room. Multiple sensors can be packaged together in a single housing so that they can be easily installed in the operating room.

An aspect of the present disclosure is to monitor various aspects of the surgical environment and the status of the OR door(s) to and to issue actionable alerts. The cameras (e.g., cameras 104a and 104b) placed inside the OR can detect whether an OR door is open or closed during surgery using one or more machine-learning models. Further, the sensors (e.g., sensors 108) placed inside the OR can detect aspects of the environment such as temperature, humidity, pressure, and air quality (e.g., particulate matter or particle count detected in the air). If an alert threshold is reached, the system can generate and issue alerts to users to take appropriate actions (e.g., closing the door). The alert threshold can include a temperature threshold (e.g., whether the OR temperature is not within a predefined range), a humidity threshold (e.g., whether the OR humidity is not within a predefined range), a pressure threshold (e.g., whether the OR pressure is not within a predefined range), an air quality threshold (e.g., whether the OR particle count is not within a predefined range), a time threshold (e.g., whether the door remains open over a predefined duration), or any combination thereof. Data gathered by the system can be stored in and/or transmitted to EMR/HIS/EHR systems to investigate environmental conditions during the surgery and identify causes for SSI.

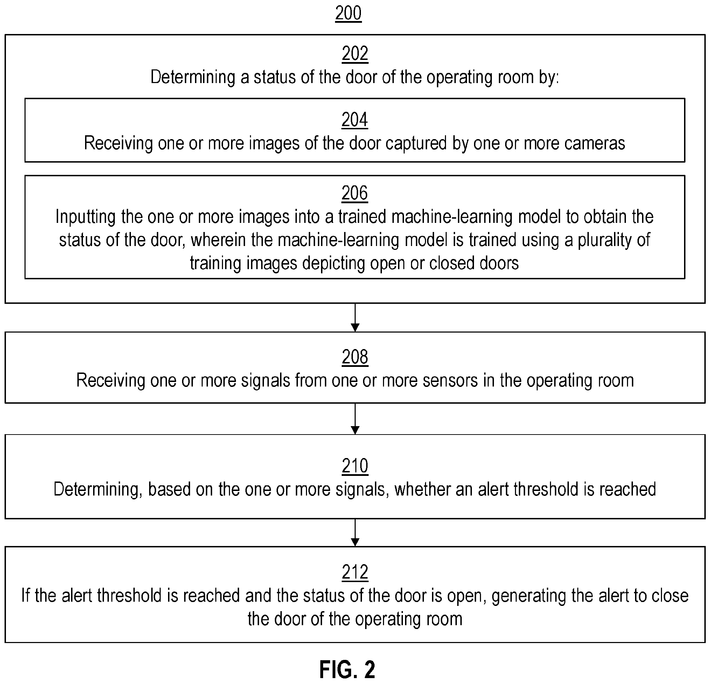

FIG. 2 illustrates an exemplary process 200 for generating an alert to close a door of an operating room, according to various examples. Process 200 is performed, for example, using one or more electronic devices implementing a software platform. Process 200 can be performed using a client-server system, and the blocks of process 200 can be divided up in any manner between the server and a client device. The blocks of process 200 can, for example, be divided up between the server and multiple client devices. Alternatively, process 200 can be performed using only a client device or only multiple client devices. In process 200, some blocks are, optionally, combined, the order of some blocks is, optionally, changed, and some blocks are, optionally, omitted. Additional steps may be performed in combination with the process 200. Accordingly, the operations as illustrated (and described in greater detail below) are exemplary by nature and, as such, should not be viewed as limiting.

At block 202, an exemplary system (e.g., one or more electronic devices) determines a status of the door of the operating room (e.g., door 102 in FIG. 1), such as whether the door is open or closed. In some examples, block 202 comprises blocks 204 and block 206, as described below.

At block 204, the system receives one or more images of the door captured by one or more cameras (e.g., cameras 104a and/or 104b in FIG. 1). As discussed above, the one or more cameras can be placed inside the operating room. The one or more cameras can be oriented toward the door such that they can capture images of the door. Multiple cameras can be placed in different locations in the operating room such as they can collectively capture the OR door from different perspectives. The one or more cameras can, for example, include PTZ cameras. The one or more cameras can, for example, include a camera integrated into a surgical light in the operating room.

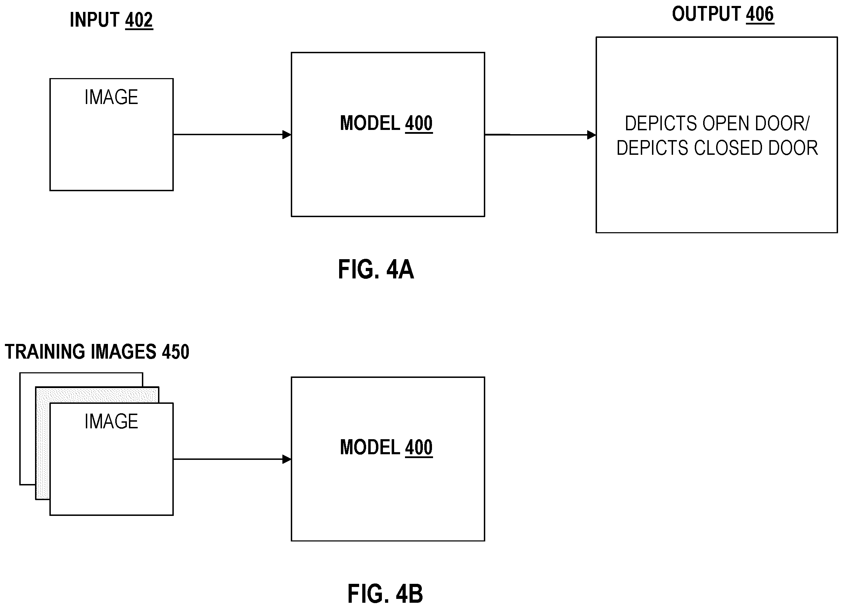

At block 206, the system inputs the one or more images into a trained machine-learning model to obtain the status of the door. FIG. 4A illustrates an exemplary machine-learning model used to determine the status of the door, in accordance with some examples. As shown, the model 400 is configured to receive an input image 402 and output an indication 406 of whether it depicts an open door or closed door. The machine-learning model 400 can be an object detection algorithm that can identify a door in the input image and further determine whether it is open or closed. The machine-learning model 400 can be a neural network model such as a convolutional neural network. It should be appreciated by one of ordinary skill in the art that other types of object detection algorithms that provide sufficient performance and accuracy in real time can be used.

The machine-learning model is trained using a plurality of training images depicting open or closed doors. FIG. 4B illustrates an exemplary training process, in accordance with some examples. In this example, the model 400 is trained using a plurality of annotated training images 450. Some or all of the annotated images 450 can depict a door and include a label indicating where the door is and whether the depicted door is open or closed. At least some of the annotated images 450 can be captured in the same operating room (e.g., operating room 100) for which the model will be deployed and can depict the same door as the door in the input image (e.g., input image 402). During training, the model receives each image of the annotated images 350 and provides an output (e.g., location of a detected door in the image, the status of the detected door). The output is compared against the annotation associated with the image. Based on the comparison, the model 400 can be updated (e.g., via a backpropagation process).

For example, the system can start monitoring the status of the OR door upon determining that a surgery is in progress. For example, upon determining that a surgery is in progress in the operating room, the system can start capturing images of the door (e.g., for analysis in blocks 204-206). As another example, the system can continuously capture images of the door, but only input the images into the trained machine-learning model for analysis in block 206 upon determining that a surgery is in progress in the operating room. On the other hand, if the system determines that a surgery is not in progress (e.g., the surgery has not started, the surgery has ended), the system can forego analyzing images of the door.

To determine whether a surgery is progress, the system can be configured to detect or track various objects of interest, such as: a patient, a stretcher, a surgical mask, an intubation mask, an anesthesia cart, a cleaning cart, an operating table, an X-Ray device (e.g., C-Arm X-Ray), an imaging device (e.g., mobile CT), a surgeon, the surgeon's hand, a scalpel, a case cart, a surgical robot, an endoscope, a trocar, an oxygen mask, a light in the operating room (e.g., a surgical light), the door, a surgical drape, or any combination thereof. To detect a particular object, the system can input one or more images into an algorithm (e.g., a trained machine-learning model) configured to detect the particular object. Once the object is detected, the system can track the movement of the objects over time using a tracking algorithm. Based on the detected and/or tracked objects, the system can determine what is occurring in the operating room, such as whether the surgery is in progress. The system can, for example, determines whether the surgery is in progress based on: whether the stretcher is brought into the operating room with the patient, whether the surgeon is masked, whether the patient is masked (e.g., oxygen mask can indicates general anesthesia induction has begun), whether the patient is draped, whether the patient is put on the operating table, whether the surgeon is donning a gown, whether the surgeon has picked up a surgical tool, whether the patient is intubated, whether the patient is on the operating table, whether an incision is made, whether the surgical light is in use, whether a medical device (e.g., X-Ray device) is in use or brought in proximity to the patient, whether the anesthesia cart is in use, whether the imaging device is in use or within a predefined proximity to the patient, whether surgical robot is in use or within a predefined proximity to the patient, whether the case cart has been brought into the operating room, whether one or more instruments from the case cart are unwrapped, whether the cleaning cart is in use (e.g., indicating that the surgery has ended), or any combination thereof.

Determining whether a surgery is in progress can comprise determining a surgical milestone. A milestone may refer to a phase or period of time during a surgical workflow (e.g., surgical phase), or a specific time point during the surgical workflow. A surgical milestone can refer to a preoperative activity, an intraoperative activity, or a postoperative activity, as discussed herein. Some surgical milestones may include specific steps (e.g., making an incision, removing an organ) of a surgery.

A surgical milestone can indicate the stage of progression through a surgical procedure or a surgical workflow. The plurality of predefined milestones can include: whether an operating room is ready, whether operating room setup has started, whether a medical staff member (e.g., the surgeon, the scrub nurse, the technician) is donning surgical attire (e.g., masks, gloves, caps, gowns), whether operating room equipment is being set up, whether the patient is brought in to the operating room, whether the patient is ready for intubation or anesthesia, whether a timeout is occurring, whether the timeout has occurred, whether the patient is intubated or anesthetized, whether the patient has been prepped and draped for surgery, whether the patient is ready for surgery, whether a surgery site prep is complete, whether a surgery has started, whether the surgery is closing, whether a dressing is applied to the patient, whether the surgery is stopped, whether the patient is brought out of the operating room, whether the operating room is being cleaned, whether the operating room is clean, or any combination thereof. It should be understood that the foregoing list of milestones is merely exemplary. There may be fewer, additional, or different predefined milestones, for instance, depending on a type of surgical procedure.

The system can use the one or more trained machine learning models to detect one or more detected objects or events, which are in turn used to determine the one or more surgical milestones (e.g., surgical time points, surgical phases). The one or more trained machine learning models can include an object detection algorithm, an object tracking algorithm, a video action detection algorithm, an anomaly detection algorithm, or any combination thereof.

The system can first use an object detection algorithm to detect a particular type of object in an image, and then use an object tracking algorithm to track the movement and/or status of the detected object in subsequent images. Using one or more object detection algorithms, the system may detect one or more objects and assign an object ID to each detected object. The one or more object detection algorithms can comprise machine-learning models such as a 2D convolutional neural network (CNN) or 3D-CNN (e.g., MobileNetV2, ResNet, MobileNetV3, CustomCNN). After the objects are detected, the system may then use one or more object tracking algorithms to track the movement of the detected objects. The one or more object tracking algorithms can comprise any computer-vision algorithms for tracking objects and can comprise non-machine-learning algorithms. The object tracking algorithm(s) may involve execution of more lightweight code than the object detection algorithm(s), thus improving efficiency and reducing latency for surgical milestone determination. The object detection algorithm can include an instance segmentation algorithm, which can be configured to simultaneously perform classification (e.g., determining what type of object an image depicts), semantic segmentation (e.g., determining what pixels in the image belong to the object), and instance association (e.g., identifying individual instances of the same class; for example, person1 and person2). Additionally, in real-world scenes, a given visual object may be occluded by other objects. Although human vision systems can locate and recognize severely occluded objects with temporal context reasoning and prior knowledge, it may be challenging for classical video understanding systems to perceive objects in the heavily occluded video scenes. Accordingly, some examples of the present disclosure include machine-learning algorithms that take into account the temporal component of the video stream. For example, the system may perform spatial feature calibration and temporal fusion for effective one-stage video instance segmentation. As another example, the system may perform spatio-temporal contrastive learning for video instance segmentation. Additional information on these exemplary algorithms can be found, for example, in Li et al., "Spatial Feature Calibration and Temporal Fusion for Effective One-stage Video Instance Segmentation," arXiv:2104.05606v1, available at https://doi.org/10.48550/arXiv.2104.05606, and Jiang et al., "STC: Spatio-Temporal Contrastive Learning for Video Instance Segmentation," arXiv:2202.03747v1, available at https://doi.org/10.48550/arXiv.2202.03747, both of which are incorporated herein by reference.

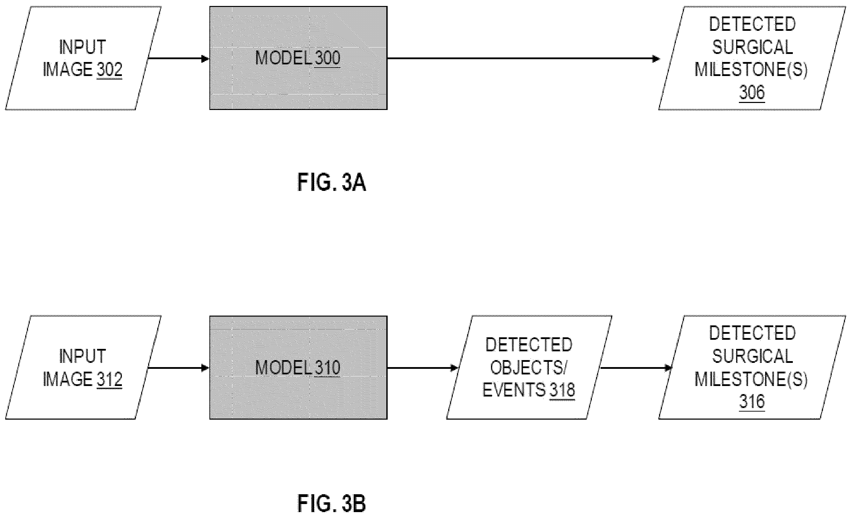

The tracked movement and/or status of one or more detected objects can then be used to determine events occurring in the operating room. For example, the system can first use an object detection model to detect a stretcher in an image and then use an object tracking algorithm to detect when the stretcher crosses door coordinates to determine that the stretcher is being moved into the operating room (i.e., an event). The one or more trained machine-learning models can be trained using a plurality of annotated images (e.g., annotated with labels of object(s) and/or event(s)). Further description of such machine learning models is provided below with reference to FIG. 3A.

An object that the system can detect can include physical items, persons, or parts thereof, located inside, entering, or leaving an operating room. The object can include a stretcher, a patient, a surgeon, an anesthesiologist, the surgeon's hand, a surgical assistant, a scrub nurse, a technician, a nurse, a scalpel, sutures, a staple gun, a door to a sterile room, a door to a non-sterile corridor, a retractor, a clamp, an endoscope, an electrocautery tool, an intubation mask, a surgical mask, a C-Arm, an Endoscopic Equipment Stack, an anesthesia machine, an anesthesia cart, a fluid management system, a waste management system, a waste disposal receptacle, an operating table, surgical table accessories, an equipment boom, an anesthesia boom, an endoscopic equipment cart, surgical lights, a case cart, a sterile back table, a sterile mayo stand, a cleaning cart, an X-Ray device, an imaging device, a trocar, a surgical drape, operating room floor, EKG leads, ECG leads, bed linens, a blanket, a heating blanket, a lap belt, safety straps, a pulse oximeter, a blood pressure machine, an oxygen mask, an IV, or any combination thereof.

An event that the system can detect can include a status, change of status, and/or an action associated with an object. The event can include whether the surgical lights are turned off, whether the operating table is vacant, whether the bed linens are wrinkled, whether the bed linens are stained, whether the operating table is wiped down, whether a new linen is applied to the operating table, whether a first sterile case cart is brought into the operating room, whether a new patient chart is created, whether instrument packs are distributed throughout the operating room, whether booms and suspended equipment are repositioned, whether the operating table is repositioned, whether a nurse physically exposes instrumentation by unfolding linen or paper, or opening instrumentation containers using a sterile technique, whether the scrub nurse entered the operating room, whether the technician entered the operating room, whether the scrub nurse is donning a gown, whether the circulating nurse is securing the scrub nurse's gown, whether the scrub nurse is donning gloves using the sterile technique, whether the sterile back table or the sterile mayo stand is being set with sterile instruments, whether the patient is wheeled into the operating room on a stretcher, whether the patient is wheeled into the operating room on a wheel chair, whether the patient walked into the operating room, whether the patient is carried into the operating room, whether the patient is transferred to the operating table, whether the patient is covered with the blanket, whether the lap belt is applied to the patient, whether the pulse oximeter is placed on the patient, whether the EKG leads are applied to the patient, whether the ECG leads are applied to the patient, whether the blood pressure cuff is applied to the patient, whether a surgical sponge and instrument count is conducted, whether a nurse announces a timeout, whether a surgeon announces a timeout, whether an anesthesiologist announces a timeout, whether activities are stopped for a timeout, whether the anesthesiologist gives the patient the oxygen mask, whether the patient is sitting and leaning over with the patient's back cleaned and draped, whether the anesthesiologist inspects the patient's anatomy with a long needle, whether the anesthesiologist injects medication into the patient's back, whether the anesthesiologist indicates that the patient is ready for surgery, whether the patient is positioned for a specific surgery, whether required surgical accessories are placed on a table, whether padding is applied to the patient, whether the heating blanket is applied to the patient, whether the safety straps are applied to the patient, whether a surgical site on the patient is exposed, whether the surgical lights are turned on, whether the surgical lights are positioned to illuminate the surgical site, whether the scrub nurse is gowning the surgeon, whether the scrub nurse is gloving the surgeon, whether skin antiseptic is applied, whether the surgical site is draped, whether sterile handles are applied to the surgical lights, whether a sterile team member is handing off tubing to a non-sterile team member, whether a sterile team member is handing off electrocautery to a non-sterile team member, whether the scalpel is handed to the surgeon, whether an incision is made, whether the sutures are handed to the surgeon, whether the staple gun is handed to the surgeon, whether the scrub nurse is handing a sponge to a sponge collection basin, whether an incision is closed, whether dressing is applied to cover a closed incision, whether the surgical lights are turned off, whether the anesthesiologist is waking the patient, whether the patient is returned to a supine position, whether extubation is occurring, whether instruments are being placed on the case cart, whether a garbage bag is being tied up, whether the bed linens are collected and tied up, whether the operating table surface is cleaned, whether the operating room floor is being mopped, whether the patient is being transferred to a stretcher, whether the patient is being brought out of the operating room, whether the surgical table is dressed with a clean linen, whether a second sterile case cart is brought into the operating room, or any combination thereof.

Instead of using trained machine-learning models to detect objects/events (which are then used to determine surgical milestones), the system can use trained machine-learning models to output surgical milestones directly. A trained machine-learning model of the one or more trained machine-learning models can be a machine-learning model (e.g., deep-learning model) trained using annotated surgical video information, where the annotated surgical video information includes annotations of at least one of the plurality of predefined surgical milestones. Further description of such machine learning models is provided below with reference to FIG. 3B.

The system may perform a spatial analysis (e.g., based on object detection/tracking as discussed above), a temporal analysis, or a combination thereof. The system may perform the temporal analysis using a temporal deep neural network (DNN), such as LSTM, Bi-LSTM, MS-TCN, etc. The DNN may be trained using one or more training videos in which the start time and the end time of various surgical milestones are bookmarked. The temporal analysis may be used to predict remaining surgery duration, as discussed below.

The one or more trained machine-learning models used herein can comprise a trained neural network model, such as a 2D CNN, 3D-CNN, temporal DNN, etc. For example, the models may comprise ResNet50, AlexNet, Yolo, I3D ResNet 50, LSTM, MSTCN, etc. The one or more trained machine-learning models may comprise supervised learning models that are trained using annotated images such as human-annotated images. Additionally, or alternatively, the one or more trained machine-learning model may comprise self-supervised learning models where a specially trained network can predict the remaining surgery duration, without relying on labeled images. As examples, a number of exemplary models are described in G. Yengera et al., "Less is More: Surgical Phase Recognition with Less Annotations through Self-Supervised Pre-training of CNN-LSTM Networks," arXiv:1805.08569 [cs.CV], available at https://arxiv.org/abs/1805.08569. For example, an exemplary model may utilize a self-supervised pre-training approach based on the prediction of remaining surgery duration (RSD) from laparoscopic videos. The RSD prediction task is used to pre-train a CNN and long short-term memory (LSTM) network in an end-to-end manner. The model may utilize all available data and reduces the reliance on annotated data, thereby facilitating the scaling up of surgical phase recognition algorithms to different kinds of surgeries. Another example model may comprise an end-to-end trained CNN-LSTM model for surgical phase recognition. It should be appreciated by one of ordinary skill in the art that other types of object detection algorithms, object tracking algorithms, video action detection algorithms, that provide sufficient performance and accuracy (e.g., in real time) can be used. The system can include machine-learning models associated with a family of architectures based on visual transformers, which may perform image recognition at scale. An exemplary framework is a Self-supervised Transformer with Energy-based Graph Optimization (STEGO) and may be capable of jointly discovering and segmenting objects without any human supervision. Building upon another self-supervised architecture, DINO, STEGO can distill pre-trained unsupervised visual features into semantic clusters using a novel contrastive loss. Additional information on visual transformers can be found, for example, in Caron et al., "An Image is Worth 16x16 Words: Transformers for Image Recognition at Scale," arXiv:2010.11929v2, available at https://doi.org/10.48550/arXiv.2010.11929, which is incorporated herein by reference. Additional information on DINO and STEGO can be found, for example, in Hamilton et al., "Unsupervised Semantic Segmentation by Distilling Feature Correspondences," arXiv:2203.08414v1, available at https://doi.org/10.48550/arXiv.2203.08414, and Caron et al., "Emerging Properties in Self-Supervised Vision Transformers," arXiv:2104.14294v2, available at https://doi.org/10.48550/arXiv.2104.14294, which are incorporated herein by reference. Additional details related to detection of surgical milestones can be found in U.S. Provisional Application entitled, "SYSTEMS AND METHODS FOR MONITORING SURGICAL WORKFLOW AND PROGRESS" (Attorney Docket No.: 16890-30044.00), which is incorporated herein by reference.

At block 208, the system receives one or more signals from one or more sensors in the operating room. The one or more sensors can include: a temperature sensor, a humidity sensor, a pressure sensor, an air quality sensor (e.g., for measuring particle counts), a gas sensor, or any combination thereof. The one or more sensors can be placed within a predefined distance from a surgery table. Various sensors may be placed in areas of interest in the operating room to improve accuracy of the monitoring. For example, one or more sensors can be placed within a predefined distance to the operating table, the door, room air vents, etc. The total number of sensors deployed in an operating room can vary based on the size of the room. For example, if the size of the room exceeds a predefined threshold, multiple sensors of the same type can be deployed to ensure accurate monitoring of the entire room. Multiple sensors can be packaged together in a single housing so that they can be easily installed in the operating room.

The system can perform block 208 upon determining that a surgery is in progress or upon determining that a particular surgical milestone is reached. For example, if the system determines that a surgery is not in progress or if a particular surgical milestone is not reached, the system may deactivate the one or more sensors, forego receiving signals from the one or more sensors, or forego processing any signals received from the one or more sensors to reduce power consumption by the sensors and/or to reduce usage of system resources (e.g., processors, memories). As another example, if the system determines that a surgery is in progress or that a particular surgical milestone is reached, the system may activate the one or more sensors, start receiving signals from the one or more sensors, or start processing signals received from the one or more sensors. The determination of surgical progress and surgical milestones is described above.

At block 210, the system determines, based on the one or more signals, whether an alert threshold is reached. The alert threshold can include a temperature threshold, a humidity threshold, a pressure threshold, an air quality threshold (e.g., a threshold for particulate matter detected in the air), or any combination thereof. The threshold can be a maximum or minimum value. The threshold can be a range (e.g., a predefined temperature range, a predefined humidity range). As an example, the system can determine that an alert threshold is reached if the current temperature is outside a predefined temperature threshold. As another example, the system can determine that an alert threshold is reached if the current humidity is outside a predefined humidity threshold. As another example, the system can determine that an alert threshold is reached if the current pressure is outside a predefined pressure threshold. As another example, the system can determine that an alert threshold is reached if the current particle count of a particular substance (e.g., a particular gas) is outside a predefined particle count threshold. As another example, the system can aggregate multiple sensor readings (e.g., current temperature, current humidity, current pressure, current air quality), for example, by calculating a weighted sum of the sensor readings, and compare the weighted sum against a predefined threshold (e.g., a single value or a range) to determine whether an alert threshold is reached.

The alert threshold can be configurable per institution or per surgery type. Some types of procedures (e.g., cardiac and orthopedic cases) are more concerned about SSIs, and thus may be associated with a different alert threshold than other surgeries (e.g., abdominal surgical cases).

The alert threshold may vary depending on the current surgical milestone. For example, the acceptable range of temperature in the operating room may vary for different surgical milestones. As another example, the acceptable range of humidity in the operating room may vary for different surgical milestones. The determination of surgical progress and surgical milestones is described above.

At block 212, if the alert threshold is reached and the status of the door is open, the system generates an alert to take an appropriate action, such as closing the door of the operating room. In some examples, the system displays the generated alert on a display in the operating room. As shown in the example in FIG. 1, an alert to close the OR door can be displayed on the display 106 in the operating room 100. In some examples, the system displays the generated alert on a display in a monitoring area. As shown in the example in FIG. 1, an alert to close the OR door of the operating room 100 can be displayed on the display 116, which can be a monitor at a central control center for monitoring multiple operating rooms. Alternatively, or additionally, the system can display the alert as a message (e.g., a text message, a notification) on a mobile device. As shown in the example in FIG. 1, an alert can be displayed on the mobile phone 114. While the alerts shown in FIG. 1 are visual alerts, it should be appreciated that the alert can additionally or alternatively comprise an audio component, a haptic component, or any combination thereof. The alert can be provided based on user-configurable settings (e.g., at a user-specified frequency).

The system can generate an alert to take an appropriate action if the alert threshold is reached, even if the door is closed. For example, a series of door openings and closings have resulted in an environmental condition exception for the operating room. The system can, for example, provide different types of alerts depending on whether the door is open or closed. If an alert threshold is reached and the status of the door is open, the system alerts the user to close the door as discussed above. On the other hand, if the alert threshold is reached and the status of the door is closed, the system foregoes providing the alert to close the door (because the door is already closed) but instead generates an environmental alert. The environmental alert can report the triggered threshold (e.g., a temperature issue, a humidity issue, a pressure issue, an air quality issue, a gas issue) such that appropriate actions can be taken. In some examples, in addition to or alternative to the environmental alert, the system can issue an alert to recommend one or more additional infection prevention protocols (e.g., increased antibiotic dose, or longer course of antibiotic treatment, or use of a different antibiotic, or use of a supplemental antibiotic). The one or more prevention protocols may be identified automatically by the system based on the case duration, the triggered alert threshold, the surgery type, etc.

Some or all of the analytics in process 200 can be displayed in a dashboard inside or outside the operating room (e.g., displays 106 and 116), including: the current door status, the time period during which the door remains open, the average duration the door is open, the triggered alert thresholds (e.g., current temperature and whether it is outside a normal range, current humidity and whether it is outside a normal range, current pressure and whether it is outside a normal range, current air quality and whether it is outside a normal range), a number of times that the door is open during the surgery, an average duration the door is open, a number of times a threshold breach occurred during a surgery, the number of times the door to the sterile room has been opened during the surgery, the number of times the door to the non-sterile corridor has been opened during the surgery, the average time the door to the sterile room remains open when it is opened, the average time the door to the non-sterile corridor remains open when it is opened, etc. If the door open counts exceed one or more configurable thresholds (which may be case duration specific or surgery type specific), the system can issue an alert to recommend some additional infection prevention protocol (e.g., increased antibiotic dose, or longer course of antibiotic treatment, or use of a different antibiotic, or use of a supplemental antibiotic.

The system can store some or all of the sensor readings over time, such as temperature measurements, humidity measurements, pressure measurements, and air quality measurements. The system can store some or all of the analytics in process 200, such as the determined door status, the determined surgery timings, the detected/tracked objects or areas of interest, the time period during which the door remains open, the generated alerts, the triggered alert thresholds, a number of times that the door is open during the surgery, an average duration the door is open, a number of times a threshold breach occurred during a surgery, the number of times the door to the sterile room has been opened during the surgery, the number of times the door to the non-sterile corridor has been opened during the surgery, the average time the door to the sterile room remains open when it is opened, the average time the door to the non-sterile corridor remains open when it is opened, etc. In some examples, the above-described information is transmitted to EMR systems (e.g., EMR system 112 in FIG. 1) for storage and/or further analysis. For example, the system can analyze the electronic medical record to determine a cause for a post-surgery complication (e.g., SSI). For example, the system can identify correlations between any of the data points above with the occurrence or severity of the complication. The system can analyze the electronic medical record to determine a recommended protocol change for future surgeries. For example, if the system determines a correlation between the duration of door opening and a post-surgery complication, the system may automatically formulate a new protocol requiring that the door remain closed for a certain period of time.

According to some aspects, details regarding the type of door (e.g., door to non-sterile corridor, door to a sterile room) are recorded in the EMR. According to some aspects, the system determines whether to issue an alert and/or what the alert should be based on the door type. For instance, it may be common for the door to the sterile room to open a few times during the surgery. But once a surgical milestone is reached (e.g., surgery has started), opening of the door to the non-sterile corridor may be considered a "never" event. Thus, the threshold for issuing an alert to close the door to the non-sterile corridor may be lower than the threshold for issuing an alert to close the door to the sterile room. For example, the system may issue an alert to close the door to the non-sterile corridor whenever it is open during the surgery, but only issue an alert to close the door to the sterile room when the door has been opened for more than a time period or a number of times and/or when the sensor readings in the operating room exceed predefined thresholds.

FIGS. 3A and 3B illustrate exemplary machine-learning models that can be used to detect surgical milestone(s), in accordance with some examples. Both models 300 and 310 can receive an input image (e.g., an image received in block 202). The model(s) 300 can be configured to directly output one or more surgical milestones depicted in the input image. In contrast, the model(s) 310 can be configured to output one or more detected objects or events 318, which in turn can be used by the system to determine one or more surgical milestones depicted in the input image. Models 300 and 310 are described in detail below.

With reference to FIG. 3A, a model 300 is configured to receive an input image 302 and directly output an output 306 indicative of one or more surgical milestones detected in the input image 302. The model 300 can be trained using a plurality of training images depicting the one or more surgical milestones. For example, the model 300 can be trained using a plurality of annotated training images. Each of the annotated images can depict a scene of an operating room and include one or more labels indicating surgical milestone(s) depicted in the scene. The plurality of annotated training images can comprise a video in which surgical milestones are bookmarked. At least some of the annotated images can be captured in the same operating room (e.g., operating room 100) for which the model will be deployed. During training, the model receives each image of the annotated images and provides an output indicative of detected surgical milestone(s). The output is compared against the labels associated with the image. Based on the comparison, the model 300 can be updated (e.g., via a backpropagation process).

With reference to FIG. 3B, a model 310 is configured to receive an input image 312 and output one or more detected objects and/or events 318 depicted in the input image 312. Based on the one or more detected objects and/or events 318, the system can determine, as output 316, one or more surgical milestones detected in the input image 312. The one or more machine learning models can be trained using a plurality of training images depicting the one or more objects and/or events. For example, the model 310 can be trained using a plurality of annotated training images. Each of the annotated images can depict a scene of an operating room and include one or more labels indicating objects and/or events depicted in the scene. At least some of the annotated images can be captured in the same operating room (e.g., operating room 100) for which the model will be deployed. During training, the model receives each image of the annotated images and provides an output indicative of one or more detected objects and/or events. The output is compared against the labels associated with the image. Based on the comparison, the model 300 can be updated (e.g., via a backpropagation process).

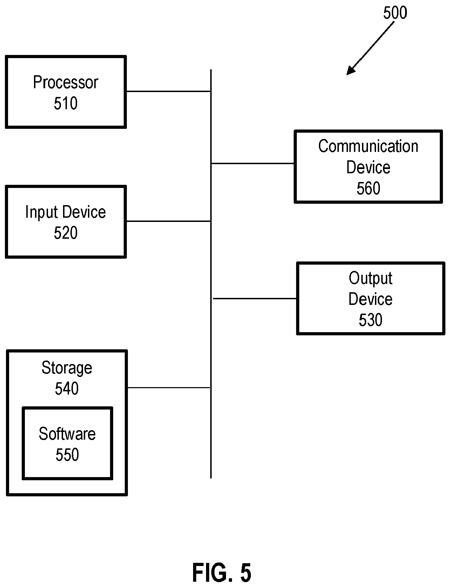

The operations described herein are optionally implemented by components depicted in FIG. 5. FIG. 5 illustrates an example of a computing device in accordance with one example. Device 500 can be a host computer connected to a network. Device 500 can be a client computer or a server. As shown in FIG. 5, device 500 can be any suitable type of microprocessor-based device, such as a personal computer, workstation, server, or handheld computing device (portable electronic device) such as a phone or tablet. The device can include, for example, one or more of processor 510, input device 520, output device 530, storage 540, and communication device 560. Input device 520 and output device 530 can generally correspond to those described above and can either be connectable or integrated with the computer.

Input device 520 can be any suitable device that provides input, such as a touch screen, keyboard or keypad, mouse, or voice-recognition device. Output device 530 can be any suitable device that provides output, such as a touch screen, haptics device, or speaker.

Storage 540 can be any suitable device that provides storage, such as an electrical, magnetic, or optical memory including a RAM, cache, hard drive, or removable storage disk. Communication device 560 can include any suitable device capable of transmitting and receiving signals over a network, such as a network interface chip or device. The components of the computer can be connected in any suitable manner, such as via a physical bus or wirelessly.

Software 550, which can be stored in storage 540 and executed by processor 510, can include, for example, the programming that embodies the functionality of the present disclosure (e.g., as embodied in the devices as described above).

Software 550 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 540, that can contain or store programming for use by or in connection with an instruction execution system, apparatus, or device.

Software 550 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate, or transport programming for use by or in connection with an instruction execution system, apparatus, or device. The transport readable medium can include, but is not limited to, an electronic, magnetic, optical, electromagnetic, or infrared wired or wireless propagation medium.

Device 500 may be connected to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T3 lines, cable networks, DSL, or telephone lines.

Device 500 can implement any operating system suitable for operating on the network. Software 550 can be written in any suitable programming language, such as C, C++, Java, or Python. In various examples, application software embodying the functionality of the present disclosure can be deployed in different configurations, such as in a client/server arrangement or through a Web browser as a Web-based application or Web service, for example.

The disclosure will now be further described by the following numbered embodiments which are to be read in connection with the preceding paragraphs, and which do not limit the disclosure. The features, options and preferences as described above apply also to the following embodiments.

Embodiment 1. A method for generating an alert to close a door of an operating room, comprising:
determining a status of the door of the operating room by:
   receiving one or more images of the door captured by one or more cameras;
   inputting the one or more images into a trained machine-learning model to obtain the status of the door, wherein the machine-learning model is trained using a plurality of training images depicting open or closed doors;
receiving one or more signals from one or more sensors in the operating room;
determining, based on the one or more signals, whether an alert threshold is reached; and
if the alert threshold is reached and the status of the door is open, generating the alert to close the door of the operating room.

Embodiment 2. The method of Embodiment 1, further comprising:
receiving a video stream captured by the one or more cameras; and
determining, based on at least a portion of the video stream, whether a surgery is in progress;
   in accordance with a determination that the surgery is in progress, starting the determination of the status of the door of the operating room;
   in accordance with a determination that the surgery is not in progress, foregoing determining the status of the door of the operating room.

Embodiment 3. The method of Embodiment 2, wherein determining whether the surgery is in progress comprises:
detecting one or more objects in the video stream; and
determining whether the surgery is in progress based on the tracked one or more objects.

Embodiment 4. The method of Embodiment 3, wherein the one or more objects include:
a stretcher,
a patient,
a surgical mask,
an intubation mask,
an anesthesia cart,
a cleaning cart,
an operating table,
an X-Ray device,
an imaging device,
a surgeon,
the surgeon's hand,
a scalpel,
an endoscope,
a trocar,
an oxygen mask,
a light in the operating room,
the door,
a surgical drape,
a case cart,
a surgical robot,
or any combination thereof.

Embodiment 5. The method of Embodiment 4, wherein determining whether the surgery is in progress is based on:
whether the stretcher is brought into the operating room,
whether the surgeon is masked,
whether the patient is masked,
whether the patient is draped,
whether the surgeon is donning a gown,
whether the patient is intubated,
whether the patient is on the operating table,
whether an incision is made,
whether the surgical light is in use,
whether the X-Ray device is in use,
whether the anesthesia cart is in use,
whether the imaging device is in use or within a predefined proximity to the patient,
whether the case cart has been brought into the operating room,
whether one or more instruments from the case cart are unwrapped,
whether the cleaning cart is in use,
or any combination thereof.

Embodiment 6. The method of any of Embodiments 1-5, wherein the trained machine-learning model is an object detection algorithm.

Embodiment 7. The method of Embodiment 6, wherein the trained machine-learning model is a neural network model.

Embodiment 8. The method of Embodiment 6 or Embodiment 7, wherein the machine-learning model is trained using a plurality of annotated images.

Embodiment 9. The method of any of Embodiments 1-8, wherein the one or more sensors include: a temperature sensor, a humidity sensor, a pressure sensor, an air quality sensor, a gas sensor, or any combination there.

Embodiment 10. The method of any of Embodiments 1-9, wherein the one or more sensors are placed within a predefined distance from a surgery table.

Embodiment 11. The method of any of Embodiments 1-10, wherein the alert threshold includes a temperature threshold, a humidity threshold, a pressure threshold, an air quality threshold, or any combination thereof.

Embodiment 12. The method of any of Embodiments 1-11, wherein the one or more cameras include a camera integrated into a surgical light.

Embodiment 13. The method of any of Embodiments 1-12, further comprising: displaying the generated alert on a display in the operating room.

Embodiment 14. The method of any of Embodiments 1-13, further comprising: displaying the generated alert on a display in a monitoring area.

Embodiment 15. The method of any of Embodiments 1-14, further comprising: displaying the alert as a message on a mobile device.

Embodiment 16. The method of any of Embodiments 1-15, further comprising: storing the determined door status, an amount of time that the door is open during a surgery, a number of times that the door is open during the surgery, an average duration the door is open, a number of times a threshold breach occurred during a surgery, and/or the one or more signals as part of an electronic medical record.

Embodiment 17. The method of Embodiment 16, further comprising: analyzing the electronic medical record to determine a cause for a post-surgery complication.

Embodiment 18. The method of Embodiment 16, further comprising: analyzing the electronic medical record to determine a recommended protocol change for future surgeries.

Embodiment 19. The method of any of Embodiments 1-18, wherein the door of the operating room is to a non-sterile corridor where patient enters/exits through.

Embodiment 20. The method of any of Embodiments 1-18, wherein the door of the operating room is to a sterile room where sterile equipment and staff enter/exit through.

Embodiment 21. The method of any of Embodiments 1-20, further comprising: if the alert threshold is reached and the status of the door is closed:
foregoing generating the alert to close the door of the operating room; and
generating an environmental alert.

Embodiment 22. A system for generating an alert to close a door of an operating room, comprising:
one or more processors;
a memory; and
one or more programs, wherein the one or more programs are stored in the memory and configured to be executed by the one or more processors, the one or more programs including instructions for:
   determining a status of the door of the operating room by:
      receiving one or more images of the door captured by one or more cameras;
      inputting the one or more images into a trained machine-learning model to obtain the status of the door, wherein the machine-learning model is trained using a plurality of training images depicting open or closed doors;
   receiving one or more signals from one or more sensors in the operating room;
   determining, based on the one or more signals, whether an alert threshold is reached; and
   if the alert threshold is reached and the status of the door is open, generating the alert to close the door of the operating room.

Embodiment 23. The system of Embodiment 22, wherein the one or more programs further comprise instructions for:
receiving a video stream captured by the one or more cameras; and
determining, based on at least a portion of the video stream, whether a surgery is in progress;
   in accordance with a determination that the surgery is in progress, starting the determination of the status of the door of the operating room;
   in accordance with a determination that the surgery is not in progress, foregoing determining the status of the door of the operating room.

Embodiment 24. The system of Embodiment 23, wherein determining whether the surgery is in progress comprises:
detecting one or more objects in the video stream; and
determining whether the surgery is in progress based on the tracked one or more objects.

Embodiment 25. The system of Embodiment 24, wherein the one or more objects include:
a stretcher,
a patient,
a surgical mask,
an intubation mask,
an anesthesia cart,
a cleaning cart,
an operating table,
an X-Ray device,
an imaging device,
a surgeon,
the surgeon's hand,
a scalpel,
a case cart,
a surgical robot,
an endoscope,
a trocar,
an oxygen mask,
a light in the operating room,
the door,
a surgical drape,
or any combination thereof.

Embodiment 26. The system of Embodiment 25, wherein determining whether the surgery is in progress is based on:
whether the stretcher is brought into the operating room,
whether the surgeon is masked,
whether the patient is masked,
whether the patient is draped,
whether the surgeon is donning a gown,
whether the patient is intubated,
whether the patient is on the operating table,
whether an incision is made,
whether the surgical light is in use,
whether the X-Ray device is in use,
whether the anesthesia cart is in use,
whether the imaging device is in use or within a predefined proximity to the patient,
whether the cleaning cart is in use,
whether the case cart has been brought into the operating room,
whether one or more instruments from the case cart are unwrapped,
or any combination thereof.

Embodiment 27. The system of any of Embodiments 22-26, wherein the trained machine-learning model is an object detection algorithm.

Embodiment 28. The system of Embodiment 27, wherein the trained machine-learning model is a neural network model.

Embodiment 29. The system of Embodiment 27 or Embodiment 28, wherein the machine-learning model is trained using a plurality of annotated images.

Embodiment 30. The system of any of Embodiments 22-29, wherein the one or more sensors include: a temperature sensor, a humidity sensor, a pressure sensor, an air quality sensor, a gas sensor, or any combination there.

Embodiment 31. The system of any of Embodiments 22-30, wherein the one or more sensors are placed within a predefined distance from a surgery table.

Embodiment 32. The system of any of Embodiments 22-31, wherein the alert threshold includes a temperature threshold, a humidity threshold, a pressure threshold, an air quality threshold, or any combination thereof.

Embodiment 33. The system of any of Embodiments 22-32, wherein the one or more cameras include a camera integrated into a surgical light.

Embodiment 34. The system of any of Embodiments 22-33, wherein the one or more programs further comprise instructions for: displaying the generated alert on a display in the operating room.

Embodiment 35. The system of any of Embodiments 22-34, wherein the one or more programs further comprise instructions for: displaying the generated alert on a display in a monitoring area.

Embodiment 36. The system of any of Embodiments 22-35, wherein the one or more programs further comprise instructions for: displaying the alert as a message on a mobile device.

Embodiment 37. The system of any of Embodiments 22-36, wherein the one or more programs further comprise instructions for: storing the determined door status, an amount of time that the door is open during a surgery, a number of times that the door is open during the surgery, an average duration the door is open, a number of times a threshold breach occurred during a surgery, and/or the one or more signals as part of an electronic medical record.

Embodiment 38. The system of Embodiment 37, wherein the one or more programs further comprise instructions for: analyzing the electronic medical record to determine a cause for a post-surgery complication.

Embodiment 39. The system of Embodiment 37, wherein the one or more programs further comprise instructions for: analyzing the electronic medical record to determine a recommended protocol change for future surgeries.

Embodiment 40. The system of any of Embodiments 22-39, wherein the door of the operating room is to a non-sterile corridor where patient enters/exits through.

Embodiment 41. The system of any of Embodiments 22-39, wherein the door of the operating room is to a sterile room where sterile equipment and staff enter/exit through.

Embodiment 42. The system of any of Embodiments 22-41, wherein the one or more programs further comprise instructions for: if the alert threshold is reached and the status of the door is closed:
foregoing generating the alert to close the door of the operating room; and
generating an environmental alert.

Embodiment 43. A non-transitory computer-readable storage medium storing one or more programs, the one or more programs comprising instructions, which when executed by one or more processors of an electronic device, cause the electronic device to perform any of the methods of Embodiments 1-21.

Although the disclosure and examples have been fully described with reference to the accompanying figures, it is to be noted that various changes and modifications will become apparent to those skilled in the art. Such changes and modifications are to be understood as being included within the scope of the disclosure and examples as defined by the claims.

The foregoing description, for purpose of explanation, has been described with reference to specific examples. However, the illustrative discussions above are not intended to be exhaustive or to limit the invention to the precise forms disclosed. Many modifications and variations are possible in view of the above teachings. The examples were chosen and described in order to best explain the principles of the techniques and their practical applications. Others skilled in the art are thereby enabled to best utilize the techniques and various examples with various modifications as are suited to the particular use contemplated.

## Claims

1. A method for generating an alert to close a door of an operating room, comprising:
determining a status of the door of the operating room by:
receiving one or more images of the door captured by one or more cameras;
inputting the one or more images into a trained machine-learning model to obtain the status of the door, wherein the machine-learning model is trained using a plurality of training images depicting open or closed doors;
receiving one or more signals from one or more sensors in the operating room;
determining, based on the one or more signals, whether an alert threshold is reached; and
if the alert threshold is reached and the status of the door is open, generating the alert to close the door of the operating room.

2. The method of claim 1, further comprising:
receiving a video stream captured by the one or more cameras; and
determining, based on at least a portion of the video stream, whether a surgery is in progress;
in accordance with a determination that the surgery is in progress, starting the determination of the status of the door of the operating room;
in accordance with a determination that the surgery is not in progress, foregoing determining the status of the door of the operating room;
wherein optionally determining whether the surgery is in progress comprises:
detecting one or more objects in the video stream; and
determining whether the surgery is in progress based on the detected one or more objects.

3. The method of claim 2, wherein the one or more objects include:
a stretcher,
a patient,
a surgical mask,
an intubation mask,
an anesthesia cart,
a cleaning cart,
an operating table,
an X-Ray device,
an imaging device,
a surgeon,
the surgeon's hand,
a scalpel,
an endoscope,
a trocar,
an oxygen mask,
a light in the operating room,
the door,
a surgical drape,
a case cart,
a surgical robot,
or any combination thereof;
wherein optionally determining whether the surgery is in progress is based on:
whether the stretcher is brought into the operating room,
whether the surgeon is masked,
whether the patient is masked,
whether the patient is draped,
whether the surgeon is donning a gown,
whether the patient is intubated,
whether the patient is on the operating table,
whether an incision is made,
whether the surgical light is in use,
whether the X-Ray device is in use,
whether the anesthesia cart is in use,
whether the imaging device is in use or within a predefined proximity to the patient,
whether the case cart has been brought into the operating room,
whether one or more instruments from the case cart are unwrapped,
whether the cleaning cart is in use,
or any combination thereof.

4. The method of any of claims 1-3, wherein the trained machine-learning model is an object detection algorithm;
wherein optionally the trained machine-learning model is a neural network model; and/or
wherein optionally the machine-learning model is trained using a plurality of annotated images.

5. The method of any of claims 1-4, wherein the one or more sensors include: a temperature sensor, a humidity sensor, a pressure sensor, an air quality sensor, a gas sensor, or any combination there; and/or
wherein the one or more sensors are placed within a predefined distance from a surgery table.

6. The method of any of claims 1-5, further comprising: displaying the generated alert
(a) on a display in the operating room,
(b) on a display in a monitoring area, and/or
(c) as a message on a mobile device.

7. The method of any of claims 1-6, further comprising: storing the determined door status, an amount of time that the door is open during a surgery, a number of times that the door is open during the surgery, an average duration the door is open, a number of times a threshold breach occurred during a surgery, and/or the one or more signals as part of an electronic medical record;
optionally further comprising: analyzing the electronic medical record to determine a cause for a post-surgery complication, and/or to determine a recommended protocol change for future surgeries.

8. The method of any of claims 1-7, further comprising: if the alert threshold is reached and the status of the door is closed:
foregoing generating the alert to close the door of the operating room; and
generating an environmental alert.

9. A system for generating an alert to close a door of an operating room, comprising:
one or more processors;
a memory; and
one or more programs, wherein the one or more programs are stored in the memory and configured to be executed by the one or more processors, the one or more programs including instructions for:
determining a status of the door of the operating room by:
receiving one or more images of the door captured by one or more cameras;
inputting the one or more images into a trained machine-learning model to obtain the status of the door, wherein the machine-learning model is trained using a plurality of training images depicting open or closed doors;
receiving one or more signals from one or more sensors in the operating room;
determining, based on the one or more signals, whether an alert threshold is reached; and
if the alert threshold is reached and the status of the door is open, generating the alert to close the door of the operating room.

10. The system of claim 9, wherein the one or more programs further comprise instructions for:
receiving a video stream captured by the one or more cameras; and
determining, based on at least a portion of the video stream, whether a surgery is in progress;
in accordance with a determination that the surgery is in progress, starting the determination of the status of the door of the operating room;
in accordance with a determination that the surgery is not in progress, foregoing determining the status of the door of the operating room;
wherein optionally determining whether the surgery is in progress comprises:
detecting one or more objects in the video stream; and
determining whether the surgery is in progress based on the tracked one or more objects.

11. The system of any of claims 9-10, wherein the trained machine-learning model is an object detection algorithm;
wherein optionally the trained machine-learning model is a neural network model; and/or
wherein optionally the machine-learning model is trained using a plurality of annotated images.

12. The system of any of claims 9-11, wherein the one or more sensors include: a temperature sensor, a humidity sensor, a pressure sensor, an air quality sensor, a gas sensor, or any combination there, and/or
wherein the one or more sensors are placed within a predefined distance from a surgery table;
wherein optionally the alert threshold includes a temperature threshold, a humidity threshold, a pressure threshold, an air quality threshold, or any combination thereof.

13. The system of any of claims 9-12, wherein the one or more cameras include a camera integrated into a surgical light.

14. The system of any of claims 9-13, wherein the one or more programs further comprise instructions for: storing the determined door status, an amount of time that the door is open during a surgery, a number of times that the door is open during the surgery, an average duration the door is open, a number of times a threshold breach occurred during a surgery, and/or the one or more signals as part of an electronic medical record;
wherein the one or more programs optionally further comprise instructions for: analyzing the electronic medical record to determine a cause for a post-surgery complication, and/or for: analyzing the electronic medical record to determine a recommended protocol change for future surgeries.

15. A non-transitory computer-readable storage medium storing one or more programs, the one or more programs comprising instructions, which when executed by one or more processors of an electronic device, cause the electronic device to perform any of the methods of claims 1-8.
